# EUROPEAN PATENT SPECIFICATION

(11) **EP 1 603 620 B1**
(45) Date of publication and mention of the grant of the patent: **24.12.2008**
(21) Application number: 04704103.3
(22) Date of filing: 21.01.2004
(51) Int. Cl.: A61M 25/00, A61B 8/12, A61B 1/227, G01B 11/02, G01B 11/24

(54) **DETERMINING THE GEOMETRY AND DIMENSIONS OF A THREE-DIMENSIONAL OBJECT**
BESTIMMUNG DER GEOMETRIE UND ABMESSUNGEN EINES DREIDIMENSIONALEN GEGENSTANDS
DETERMINATION DE LA GEOMETRIE ET DES DIMENSIONS D'UN OBJET TRIDIMENSIONNEL

(30) Priority: 11.03.2003 US 385587
(43) Date of publication of application: 14.12.2005
(73) Proprietor: SIEMENS HEARING INSTRUMENTS, INC., Piscataway, NJ 08855-1397 (US)
(72) Inventor: MASTERS, Martin W., Hillsborough, New Jersey 08844 (US)
(74) Representative: Clarke, Alison Clare
(86) International application number: PCT/US2004/001623
(87) International publication number: WO 2004/081492

(56) References cited:
- WO-A-00/34739
- WO-A-02/16865
- WO-A-02/21894
- BE-A- 1 010 200
- US-A- 6 004 271
- US-A- 6 162 179

## Description

### Background and Summary of the Invention

A hearing instrument residing partially or wholly in the ear canal of the user requires a shell that can be comfortably inserted and retained in the ear canal. Currently, a variety of labor-intensive techniques such as wax molds are employed to obtain a three-dimensional geometry of the ear canal. Given the increasing use of rapid prototyping and manufacturing technology to fabricate the hearing instrument's shell, an electronic scanning technique that directly yields a digital representation of the ear canal and any desired surrounding structure, or perhaps a representation of the outer ear itself, would be most desirable.

Such a digital representation of the ear canal and the outer ear may be obtained with an apparatus having a flexible distance-measuring or imaging assembly such as an imaging catheter or an endoscopic unit that travels within a lumen of known geometry and size. This apparatus is Inserted In the ear canal and then a series of measuring or imaging processes are performed as the assembly is repositioned axially, and perhaps rotationally within the apparatus. The output of the assembly is a set of distances that are correlated with the geometry and dimensions of the lumen to reconstruct the geometry of the scanned area.

BE-A-1 010 200 discloses a device for producing earpieces- The device includes a probe comprising a fine pin that Is provided with various sensors. Scanning takes place by pushing the pin into the auditory duct. A 2D measurement is obtained by rotating the probe, and a 3D measurement is obtained by displacing the probe in the longitudinal direction z.

The invention is defined by the apparatus of claim 1 and by the methods of claim 8 and respectively 10.

### Brief Description of the Drawings

FIG. 1 is a drawing of an ear and an ear canal with an apparatus for determining the surrounding geometry and dimensions of the inserted therein;
FIG. 2 is a drawing of a section of the ear canal with the apparatus inserted therein;
FIG. 3 is a drawing of an apparatus and a device for determining the three-dimensional geometry and dimensions of the lumen of the apparatus: and
Figure 4 is a drawing of an apparatus that can produce a three-dimensional representation of an object such as teeth.

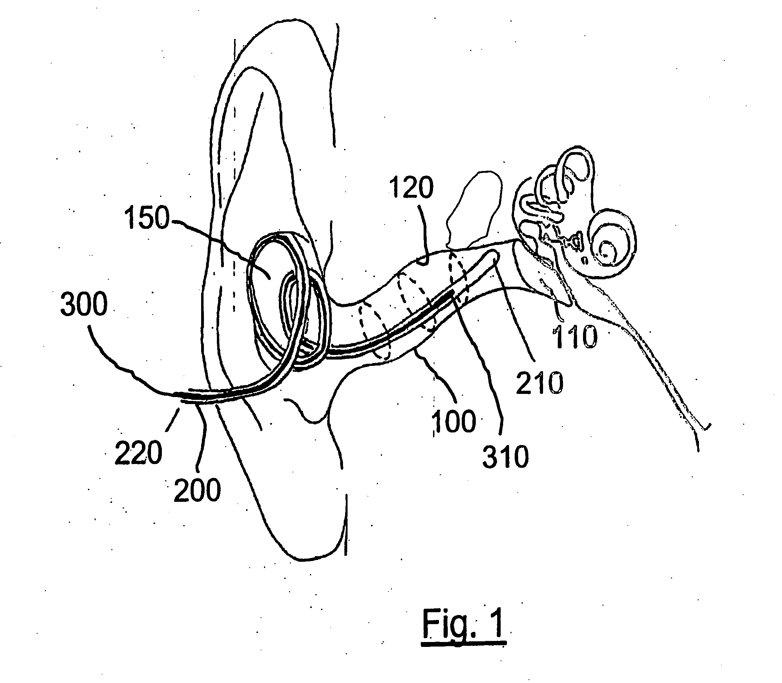

A cross-section of an ear and ear canal 100 is illustrated in Figure 1. An apparatus 200 having a lumen 220 of known and fixed geometry and dimensions is positioned in the ear canal 100, its tip 210 nearly reaching the tympanic membrane 110. The apparatus 200 may be fabricated from an optically-clear, rigid or semi-rigid, bio-compatible material, such as polycarbonate. A distance-measuring or imaging assembly 300, such as an imaging catheter or an endoscopic unit (hereafter the "imaging assembly 300"), is inserted into the lumen 220, and travels within and through the lumen 220. In addition to axial freedom of motion, the imaging assembly 300 may also rotate within the lumen 220.

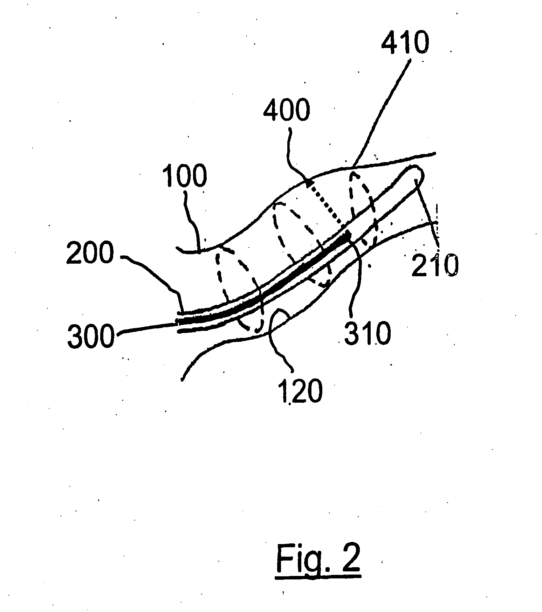

Using a method and apparatus such as that described in U.S. Patent No. 6,134,003, incorporated herein by reference, the imaging assembly 300 performs a measuring or imaging function, generating an output proportional to the distance from the assembly tip 310 (the point of measurement) to a point 400 on the inner wall 120 of the ear canal 100 (see Figure 2). Since the geometry and dimensions of the lumen 220 and the position of the assembly tip 310 within the lumen 220 are known, the position of the point 400 relative to the tip 310 can be determined from the output of the assembly 300.

To reconstruct the geometry and dimensions of the ear canal 100, a set of points along the length the inner wall 120 are required. If desired, the process can continue into the concha or bowl 150 of the ear, the portion of the ear outside of the ear canal 100. Referring to Figure 2, the tip 310 of the assembly 300 is shown at a position a distance away from the apparatus tip 210. Given the particular rotational orientation of the assembly 300 shown in the figure, the imaging assembly 300 measures (or images) the distance to a point 400 on the inner wall 120 of the ear canal 100. If the imaging assembly 300 is rotated, a series of points 410 around the inner wall 120 will be generated. Note that the points 400 comprising the series 410 need not define a closed path but rather, if the imaging assembly 300 is withdrawn simultaneously as it is rotated, the path 410 would be helical or spiral in form.

The imaging assembly 300 may use ultrasound, optical coherence tomography, or any other suitable technology for determining the distance from the assembly 300 to inner wall 120 of the ear canal 100. The catheters described in U.S. Patent No. 6,134,003 and No. 5,830,145, also incorporated herein by reference, are suitable for use in the imaging assembly 300.

The set of data points for the entire ear canal 100 and optionally at least a portion of the concha 150 is obtained by repositioning the imaging assembly 300 within the lumen 220 after each measuring or imaging process. This repositioning may be achieved by moving the imaging assembly 300 axially after each measurement and perhaps rotating the imaging assembly 300 as well. The assembly 300 could be pulled out of the lumen 220, either step-wise or continuously, resulting in either sliced or helical data. The number of points measured or imaged will determine the resolution achieved and interpolation may be utilized to smooth the data and provide a continuous surface.

The raw data from the imaging assembly 300 is the distance from the tip 310 to the inner wall 120 measured orthogonally at a number of points along the length of the lumen 220. When the rotational orientation of the imaging assembly 300 is coupled with the previously-known three-dimensional geometry and dimensions of the lumen 220 (and therefore the relative location of the assembly tip 310 at any point within the lumen 220), each distance measurement can be converted to a point in space (i.e., in xyz coordinates or any other suitable coordinate system, the origin perhaps being the apparatus tip 210). The conversion can be performed manually or with the aid of a computer program.

The resulting point data in turn may be processed to remove outliers and other unwanted information, such as noise. The remaining data represents a three-dimensional image of the ear canal, concha, and other anatomy, complete or in part as desired. This in turn may be supplied to a rapid prototyping method such as that described in U.S. Patent Application no. 09/887,939, filed June 22, 2001, incorporated by reference herein.

The apparatus 200 may have a circular cross-section and may be provided in a variety of sizes and shapes to accommodate different ears. The tip 210 may be closed or open, and the lumen 220 may have straight, bent, spiral, or curved (circular, elliptical, parabolic, or other) sections. Additionally, the apparatus 200 itself (providing a framework for the lumen 220) may have straight, bent, spiral, or curved (circular, elliptical, parabolic, or other) sections, following the shape of the lumen 220.

Instead of using a rigid material for the apparatus 200, the apparatus 200 may be fabricated from a semi-rigid material shaped for the patient's ear. Also, the apparatus 200 could have more than one lumen 220, allowing it to carry more than one imaging assembly 300.

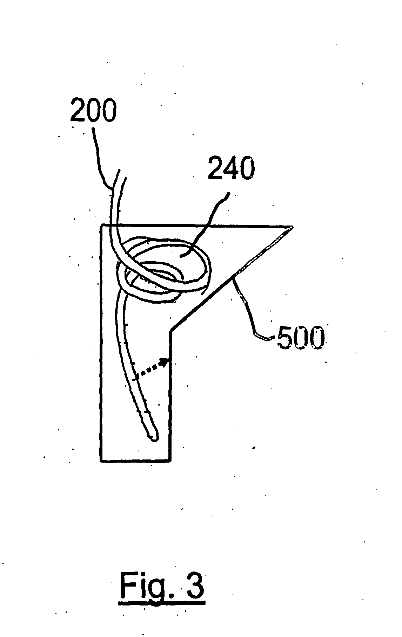

The three-dimensional geometry and dimensions of the lumen 220 may be determined by measuring or imaging an object of known dimensions. For example, the ear canal and the adjacent outer ear might be approximated as a cylinder and a cone (or a truncated cone) attached thereto, respectively. Such an object 500 is shown in Figure 3. Since the points on the surface of the object 500 may be defined as a set of predetermined xyz coordinates, by working backwards using the distance and the radial orientation of the imaging assembly 300, the geometry and dimensions of the lumen 220 can be determined.

Given the shape of lumen 220, data may be generated for points within the spiral portion 240 of the lumen 220 if the imaging assembly 300 rotates a full 360° and the measuring or imaging continues in that region. In that case, those data points within the spiral portion 240 can be discarded. Alternatively, data collection could cease beyond a predetermined arc of rotation or in the event of a discontinuity in the reflections, which would arise as the reflections sensed by the imaging assembly 300 passes from reflecting off the concha or bowl 150 of the ear (that portion of the ear external to the ear canal 100). As an additional alternative, the assembly 300 could be rotated only a portion of the entire 360°.

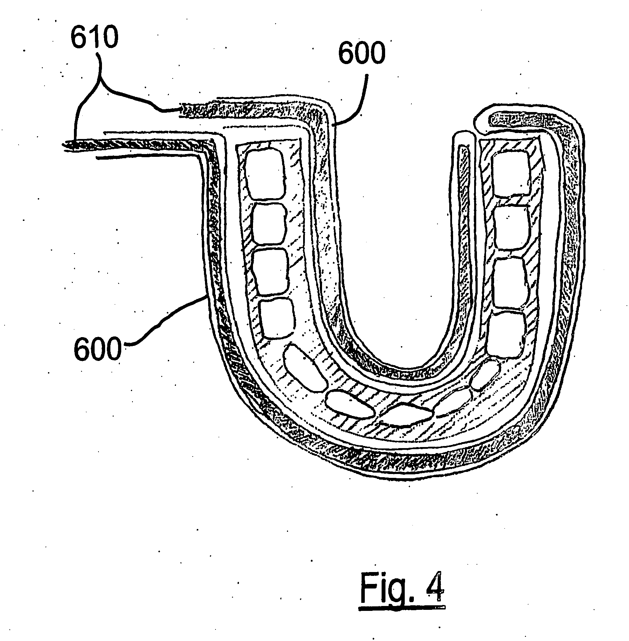

This method of determining geometry and dimensions may be used with any object. Given a set of known points from which measurements are taken, one can determine the three-dimensional geometry and size of an object. In the case of the ear canal discussed, above, the set of known points is obtained with a lumen 220 of known geometry and dimensions. Other objects, such as teeth could also be measured or imaged, given an appropriate device or apparatus containing a channel or lumen for holding and routing the distance-measuring or imaging assemblies. A partial cross-section of such a apparatus 600 is shown in Figure 4. The apparatus is provided with two assemblies 610 for measuring or imaging the two sides of the semi-circular row of teeth.

## Claims

1. An apparatus, comprising:
at least one lumen of known, fixed geometry and dimensions, contained within an optically-clear material;
a distance-measuring or imaging assembly that travels axially within the lumen and performs measuring or imaging operations at one or more points within the lumen;
means for repositioning the distance-measuring or imaging assembly axially within the lumen;
means for enabling the distance-measuring or imaging assembly to perform measuring or imaging operations as the distance-measuring or imaging assembly is positioned at one or more points within the lumen;
means for determining the relative position of the distance-measuring or imaging assembly within the lumen; **characterized in that** it further comprises:
means for correlating the relative positions of the distance-measuring or imaging assembly in the lumen with the distance-measuring or imaging operations; and
means, responsive to the means for correlating the relative positions of the distance-measuring or imaging assembly, for determining the positions of the distance-measuring or imaging assembly in space, the means for determining the position comprising means for correlating the distance-measuring or imaging operations and the respective relative positions of the distance-measuring or imaging assembly with the know, fixed three-dimensional geometry and dimensions of the lumen.

2. An apparatus as set forth in claim 1, where the assembly has axial and rotational freedom of motion.

3. An apparatus as set forth in claim 1, where the assembly comprises an imaging catheter or an endoscopic unit.

4. An apparatus as set forth in claim 1, where at least a portion of the lumen has a straight section, a bent section, a section having a curvature, or a section spiral in form.

5. An apparatus as set forth in claim 4, where at least a portion of the apparatus has a straight section, a bent section, a section having a curvature, or a section spiral in form.

6. An apparatus as set forth in claim 1, where the apparatus comprises two or more lumens, each lumen comprising a distance-measuring or imaging assembly traveling axially therein.

7. An apparatus as set forth in claim 1, where the lumen is rigid or semi-rigid.

8. A method for obtaining a three-dimensional digital representation of at least a portion of an object, comprising:
inserting an apparatus into the object, the apparatus comprising at least one lumen of known, fixed geometry and dimensions, contained within an optically-clear material; and a distance-measuring or imaging assembly that travels axially within the lumen and performs measuring or imaging operations at one or more points within the lumen;
repositioning the assembly axially within the lumen;
enabling the distance-measuring or imaging assembly to perform measuring or imaging operations as the distance-measuring or imaging assembly is positioned at one or more points within the lumen;
determining the relative position of the distance-measuring or imaging assembly within the lumen; **characterised in that** it is further comprising:
correlating the relative positions of the distance-measuring or imaging assembly in the lumen with the distance-measuring or imaging operations; and
in response to correlating the relative positions of the distance-measuring or imaging assembly, determining the positions of the distance-measuring or imaging assembly in space, where determining the positions of the distance-measuring or imaging assembly in space comprises correlating the distance-measuring or imaging operations and the respective relative positions of the distance-measuring or imaging assembly with the known, fixed three-dimensional geometry and dimensions of the lumen.

9. A method as set forth in claim 8, where the step of repositioning the assembly within the lumen comprises
moving the catheter axially within the lumen; and
optionally rotating the catheter within the lumen.

10. A method for determining the three-dimensional geometry and dimensions of a lumen contained within an optically-clear material, within an apparatus, **characterised in that** it is comprising:
inserting a distance-measuring or imaging assembly into the lumen;
inserting the apparatus into an object of known geometry and dimensions;
rotatably withdrawing the assembly from the lumen;
performing measuring or imaging operations as the assembly is withdrawn;
generating an output from the assembly and correlating the output with the known geometry and dimensions of the object; and
calculating the geometry and dimensions of the lumen.

## Patentansprüche

1. Vorrichtung, umfassend:
mindestens einen Hohlraum mit bekannten, festliegenden Formen und Abmessungen, der innerhalb eines optisch durchlässigen Materials enthalten ist;
eine Entfernungsmess- oder Bildgeberbaugruppe, die sich axial innerhalb des Hohlraums bewegt und Messungen bzw. Bildgebungsoperationen an einem oder mehreren Punkten innerhalb des Hohlraums vornimmt;
ein Mittel zum Verändern der axialen Position der Entfernungsmess- oder Bildgeberbaugruppe innerhalb des Hohlraums;
ein Mittel, das die Entfernungsmess- oder Bildgeberbaugruppe in Gang setzt, damit sie Messungen bzw. Bildgebungsoperationen vornimmt, wenn die Entfernungsmess- oder Bildgeberbaugruppe an einem oder mehreren Punkten innerhalb des Hohlraums angeordnet wird;
ein Mittel zum Bestimmen der relativen Position der Entfernungsmess- oder Bildgeberbaugruppe innerhalb des Hohlraums; **dadurch gekennzeichnet, dass** die Vorrichtung zudem umfasst:
ein Mittel zum Korrelieren der relativen Positionen der Entfernungsmess- oder Bildgeberbaugruppe in dem Hohlraum mit den Entfernungsmessungs- oder Bildgebungsoperationen; und
ein Mittel, das auf das Mittel zum Korrelieren der relativen Positionen der Entfernungsmess- oder Bildgeberbaugruppe anspricht und die Positionen der Entfernungsmess- oder Bildgeberbaugruppe im Raum bestimmt, wobei das Positionsbestimmungsmittel ein Mittel zum Korrelieren der Entfernungsmessungs- oder Bildgebungsoperationen und der jeweiligen relativen Positionen der Entfernungsmess- oder Bildgeberbaugruppe mit den bekannten festliegenden dreidimensionalen Formen und Abmessungen des Hohlraums umfasst.

2. Vorrichtung nach Anspruch 1, worin sich die Baugruppe in axialer Richtung frei bewegen und sich frei drehen kann.

3. Vorrichtung nach Anspruch 1, worin die Baugruppe einen Bildgebungskatheter oder eine Endoskopieeinheit umfasst.

4. Vorrichtung nach Anspruch 1, worin zumindest ein Teil des Hohlraums einen geraden Abschnitt, einen gebogenen Abschnitt, einen Abschnitt mit einer Krümmung oder einen Abschnitt in Spiralform besitzt.

5. Vorrichtung nach Anspruch 4, worin zumindest ein Teil der Vorrichtung einen geraden Abschnitt, einen gebogenen Abschnitt, einen Abschnitt mit einer Krümmung oder einen Abschnitt in Spiralform besitzt.

6. Vorrichtung nach Anspruch 1, worin die Vorrichtung zwei oder mehr Hohlräume umfasst, und jeder Hohlraum eine Entfernungsmess- oder Bildgeberbaugruppe enthält, die sich in axialer Richtung darin bewegt.

7. Vorrichtung nach Anspruch 1, worin der Hohlraum steif oder halbsteif ist.

8. Verfahren zum Gewinnen einer dreidimensionalen digitalen Darstellung mindestens eines Teils eines Objekts, umfassend:
das Einführen einer Vorrichtung in das Objekt, wobei die Vorrichtung mindestens einen Hohlraum mit bekannten, festliegenden Formen und Abmessungen aufweist, der innerhalb eines optisch durchlässigen Materials enthalten ist; und eine Entfernungsmess- oder Bildgeberbaugruppe, die sich axial innerhalb des Hohlraums bewegt und Messungen bzw. Bildgebungsoperationen an einem oder mehreren Punkten innerhalb des Hohlraums vornimmt;
das Verändern der axialen Position der Baugruppe innerhalb des Hohlraums;
das in Gang setzen der Entfernungsmess- oder Bildgeberbaugruppe, damit diese Messungen bzw. Bildgebungsoperationen vornehmen kann, wenn die Entfernungsmess- oder Bildgeberbaugruppe an einem oder mehreren Punkten innerhalb des Hohlraums angeordnet wird;
das Ermitteln der relativen Position der Entfernungsmess- oder Bildgeberbaugruppe innerhalb des Hohlraums; **dadurch gekennzeichnet, dass** das Verfahren zudem umfasst:
das Korrelieren der relativen Positionen der Entfernungsmess- oder Bildgeberbaugruppe in dem Hohlraum mit den Entfernungsmessungs- oder Bildgebungsoperationen; und
als Antwort auf das Korrelieren der relativen Positionen der Entfernungsmess- oder Bildgeberbaugruppe, das Bestimmen der Positionen der Entfernungsmess- oder Bildgeberbaugruppe im Raum, wobei das Bestimmen der Positionen der Entfernungsmess- oder Bildgeberbaugruppe im Raum das Korrelieren der Entfernungsmess- oder Bildgeberoperationen und der jeweiligen relativen Positionen der Entfernungsmess- oder Bildgeberbaugruppe mit den bekannten festliegenden dreidimensionalen Formen und Abmessungen des Hohlraums umfasst.

9. Verfahren nach Anspruch 8, wobei der Schritt des Veränderns der Position der Baugruppe innerhalb des Hohlraums umfasst
das Bewegen des Katheters innerhalb des Hohlraums in axialer Richtung; und
wahlweise das Drehen des Katheters innerhalb des Hohlraums.

10. Verfahren zum Bestimmen der dreidimensionalen Formen und Abmessungen eines Hohlraums, der in einem optisch durchlässigen Material innerhalb einer Vorrichtung enthalten ist, **dadurch gekennzeichnet, dass** das Verfahren umfasst:
das Einführen der Vorrichtung in ein Objekt mit bekannten Formen und Abmessungen;
das Zurückziehen der Baugruppe aus dem Hohlraum mit einer Drehbewegung;
das Vornehmen von Mess- oder Bildgebungsoperationen beim Zurückziehen der Baugruppe;
das Erzeugen eines Ausgangssignals der Baugruppe und das Korrelieren des Ausgangssignals mit den bekannten Formen und Abmessungen des Objekts; und
das Berechnen der Formen und Abmessungen des Hohlraums.

## Revendications

1. Appareil, comprenant :
au moins une lumière de géométrie et de dimensions fixées connues, contenue dans un matériau optiquement transparent ;
un ensemble de mesure de distance ou d'imagerie qui s'étend axialement dans la lumière et effectue des opérations de mesure ou d'imagerie en un ou plusieurs points dans la lumière ;
des moyens pour repositionner l'ensemble de mesure de distance ou d'imagerie axialement dans la lumière ;
des moyens pour permettre à l'ensemble de mesure de distance ou d'imagerie d'effectuer des opérations de mesure ou d'imagerie alors que l'ensemble de mesure de distance ou d'imagerie est positionné en un ou plusieurs points dans la lumière ;
des moyens pour déterminer la position relative de l'ensemble de mesure de distance ou d'imagerie dans la lumière ; **caractérisé en ce qu'**il comprend en outre :
des moyens pour corréler les positions relatives de l'ensemble de mesure de distance ou d'imagerie dans la lumière avec les opérations de mesure de distance ou d'imagerie ; et
des moyens, sensibles aux moyens pour corréler les positions relatives de l'ensemble de mesure de distance ou d'imagerie, pour déterminer les positions de l'ensemble de mesure de distance ou d'imagerie dans l'espace, les moyens pour déterminer les positions comprenant des moyens pour corréler les opérations de mesure de distance ou d'imagerie et les positions relatives respectives de l'ensemble de mesure de distance ou d'imagerie avec les géométrie et dimensions tridimensionnelles fixées connues de la lumière.

2. Appareil selon la revendication 1, dans lequel l'ensemble a une liberté de mouvement axial et de rotation.

3. Appareil selon la revendication 1, dans lequel l'ensemble comprend un cathéter d'imagerie ou une unité endoscopique.

4. Appareil selon la revendication 1, dans lequel au moins une partie de la lumière comporte une section droite, une section pliée, une section comportant une courbure, ou une section en spirale.

5. Appareil selon la revendication 4, dans lequel au moins une partie de l'appareil comporte une section droite, une section pliée, une section comportant une courbure, ou une section en spirale.

6. Appareil selon la revendication 1, dans lequel l'appareil comprend deux lumières ou plus, chaque lumière comprenant un ensemble de mesure de distance ou d'imagerie s'étendant axialement dans celle-ci.

7. Appareil selon la revendication 1, dans lequel la lumière est rigide ou semi-rigide.

8. Procédé pour obtenir une représentation numérique tridimensionnelle d'au moins une partie d'un objet, comprenant les étapes consistant à :
insérer un appareil dans l'objet, l'appareil comprenant :
au moins une lumière de géométrie et de dimensions fixées connues, contenue dans un matériau optiquement transparent ; et
un ensemble de mesure de distance ou d'imagerie qui s'étend axialement dans la lumière et effectue des opérations de mesure ou d'imagerie en un ou plusieurs points dans la lumière ;
repositionner l'ensemble axialement dans la lumière ;
permettre à l'ensemble de mesure de distance ou d'imagerie d'effectuer des opérations de mesure ou d'imagerie alors que l'ensemble de mesure de distance ou d'imagerie est positionné en un ou plusieurs points dans la lumière ;
déterminer la position relative de l'ensemble de mesure de distance ou d'imagerie dans la lumière ; **caractérisé en ce qu'**il comprend en outre les étapes consistant à :
corréler les positions relatives de l'ensemble de mesure de distance ou d'imagerie dans la lumière avec les opérations de mesure de distance ou d'imagerie ; et
en réponse à la corrélation des positions relatives de l'ensemble de mesure de distance ou d'imagerie, déterminer les positions de l'ensemble de mesure de distance ou d'imagerie dans l'espace, dans lequel la détermination des positions de l'ensemble de mesure de distance ou d'imagerie dans l'espace comprend la corrélation des opérations de mesure de distance ou d'imagerie et des positions relatives respectives de l'ensemble de mesure de distance ou d'imagerie avec les géométrie et dimensions tridimensionnelles fixées connues de la lumière.

9. Procédé selon la revendication 8, dans lequel l'étape de repositionnement de l'ensemble dans la lumière comprend les étapes consistant à :
déplacer le cathéter axialement dans la lumière ; et
optionnellement faire tourner le cathéter dans la lumière.

10. Procédé pour déterminer les géométrie et dimensions tridimensionnelles d'une lumière contenue dans un matériau optiquement transparent, dans un appareil, **caractérisé en ce qu'**il comprend les étapes consistant à :
insérer un ensemble de mesure de distance ou d'imagerie dans la lumière ;
insérer l'appareil dans un objet de géométrie et de dimensions connues ;
retirer en tournant l'ensemble de la lumière ;
effectuer des opérations de mesure et d'imagerie alors que l'ensemble est retiré ;
générer une sortie à partir de l'ensemble et corréler la sortie avec les géométrie et dimensions connues de l'objet ; et
calculer les géométrie et dimensions de la lumière.
